# EUROPEAN PATENT SPECIFICATION

(11) **EP 1 694 628 B1**
(45) Date of publication and mention of the grant of the patent: **31.08.2011**
(21) Application number: 04803588.5
(22) Date of filing: 07.12.2004
(51) Int. Cl.: C07C 67/00, C07C 69/16

(54) **PROCESS FOR THE MANUFACTURE OF TRIMETHYLHYDROQUINE DIALKANOATES**
VERFAHREN ZUR HERSTELLUNG VON TRIMETHYLHYDROCHINONDIALKANOATEN
PROCEDE DE FABRICATION DE TRIMETHYLHYDROQUINE-DIALCANOATES

(30) Priority: 15.12.2003 EP 03028811
(43) Date of publication of application: 30.08.2006
(73) Proprietor: DSM IP Assets B.V., 6411 TE Heerlen (NL)
(72) Inventor: BONRATH, Werner, 79115 Freiburg (DE); FORICHER, Yann, F-67000 Strasbourg (FR)
(74) Representative: Steck, Melanie
(86) International application number: PCT/EP2004/013903
(87) International publication number: WO 2005/058792

(56) References cited:
- EP-A- 0 850 910
- EP-A- 0 916 642
- WO-A-03/051812
- C. G. FROST ET AL.: "Indium Triflate: An Efficient Catalyst For Acylation Reactions" SYNLETT., no. 11, 1999, pages 1743-1744, XP002323059 DETHIEME VERLAG, STUTTGART.

## Description

The present invention is concerned with a process for the manufacture of 2,3,5-trimethylhydroquinone dialkanoates.

The products of the process are useful as reactants for the manufacture of 2,3,5-trimethylhydroquinone, itself a known valuable reactant for the manufacture of (all-rac)-α-tocopherol, the most active member of the vitamin E group.

2,3,5-Trimethylhydroquinone dialkanoates are known to be producible by reacting ketoisophorone with an acylating agent in the presence of a catalyst. Many such catalysts have been proposed in the past for this purpose, in particular protonic acids, e.g. inorganic acids as sulphuric acid; organic acids as p-toluenesulphonic acid; strongly acidic ion exchange resins; and Lewis acids as zinc chloride, boron trifluoride, antimony pentafluoride and titanium tetrachloride: see inter alia DE-OS 21 49 159, EP-A 0 916 642 and EP-A 1 028 103. The known procedures, depending on the particular catalyst used, entail certain disadvantages such as cost and lack of stability of the catalyst, use of chlorinated compounds, unsatisfying yield and formation of by-products.

In accordance with the present invention it has been found that the conversion of ketoisophorone to 2,3,5-trimethylhydroquinone dialkanoates can be advantageously accomplished by the use of indium tris(trifluoromethansulfonate as a catalyst.

Accordingly, the present invention provides a process for the manufacture of a 2,3,5-trimethylhydroquinone dialkanoates, which process comprises reacting ketoisophorone with an acylating agent in the presence of indium tris(trifluoromethansulfonate as a catalyst.

It may be present in an amount of from about 0.1 mol-% to about 2 mol-%, preferably in an amount of from about 0,1 mol-% to about 1 mol%, based on the amount of ketoisophorone. The catalyst may be added to the reaction mixture in solid form, anhydrous or hydrated (of which InCl₃ · 4 H₂O is an example). It can also be used in solution or in suspension: Then the catalyst is dissolved or suspended in the organic solvent, in which the reaction may be carried out. The concentration of such a solution or suspension is not critical. Furthermore, the catalyst tolerates acetic anhydride and other acylating agents as well as protonic solvents such as acetic acid, methanol, ethanol and water.

The acylating agent used in the process of the present invention may be any acylating agent that is conventionally used in the conversion of ketoisophorone to 2,3,5-trimethylhydroquinone dialkanoates, particularly acid anhydrides, acyl halides, and enol esters.

Examples of acid anhydrides are straight or branched chain alkanoic acid anhydrides such as acetic, propionic and butyric anhydride. Examples of acyl halides are straight or branched chain alkanoyl chlorides such as acetyl, propionyl and butyryl chloride. Finally, examples of enol esters are isopropenyl acetate and isopropenyl butyrate.

The preferred acylating agent is acetic anhydride or acetyl chloride, especially acetic anhydride.

The process of the present invention can be carried out in the presence or in the absence of a solvent. As a solvent, any inert polar or non-polar organic solvent or any mixture of two or more of such solvents can be used. In a preferred aspect of the invention, the reaction is carried out in the absence of a solvent.

Suitable classes of polar organic solvents include aliphatic and cyclic carbonates, aliphatic esters and cyclic esters (lactones), aliphatic and cyclic ketones and mixtures thereof. Preferred examples of aliphatic and cyclic carbonates are ethylene carbonate, propylene carbonate and 1,2-butylene carbonate. Preferred examples of aliphatic esters and cyclic esters (lactones) are ethyl acetate, isopropyl acetate and n-butyl acetate; and γ-butyrolactone. Preferred examples of aliphatic and cyclic ketones are acetone, diethyl ketone and isobutyl methyl ketone; and cyclopentanone and isophorone.

As suitable classes of non-polar organic solvents there may be mentioned aliphatic hydrocarbons and aromatic hydrocarbons. Preferred examples of aliphatic hydrocarbons are linear, branched or cyclic C₅- to C₁₅-alkanes. Particularly preferred are linear, branched or cyclic C₆- to C₁₀-alkanes, especially preferred are hexane, heptane, octane, cyclohexane and methylcyclohexane or mixtures thereof. Preferred examples of aromatic hydrocarbons are benzene, toluene, o-, m- and p-xylene 1,2,3-trimethylbenzene, pseudocumene, mesitylen, naphthalene and mixtures thereof.

The reaction can be effected in a single solvent phase, two-phase solvent systems comprising polar and non-polar solvents may, however, also be used. Examples of non-polar solvents in such two-phase solvent systems are the non-polar solvents named above. Examples of polar solvents in such two-phase solvent systems are the polar solvents named above. The most preferred two-phase solvent systems are mixtures of ethylene carbonate and/or propylene carbonate and hexane, heptane or octane, especially mixtures of ethylene carbonate and heptane, mixtures of propylene carbonate and octane, and mixtures of ethylene carbonate, propylene carbonate and heptane.

While the ratio of acylating agent to ketoisophorone is not narrowly critical the molar ratio of the acylating agent to ketoisophorone is suitably from about 1 : 1 to about 5 : 1, preferably from about 2 : 1 to about 3 : 1, and is most preferably about 3 : 1.

The process of the invention is conveniently carried out at temperatures from about 0°C to about 140°C, preferably at temperatures from about 25°C to about 90°C, more preferably at temperatures from about 25°C to about 70°C.

Moreover, the process is conveniently carried out under an inert gas atmosphere, preferably under gaseous nitrogen or argon.

The progress of the reaction is suitably monitored by gas chromatography (GC) and mass spectrometry (MS) of samples taken from the reaction mixture at various time intervals during the reaction.

The produced 2,3,5-trimethylhydroquinone dialkanoate can be isolated after distilling off the remaining acylating agent and the secondary product formed in the acylation, e.g. acetic acid when acetic anhydride is used as the acylating agent, by extraction of the crude product mixture with a suitable organic solvent, e.g. toluene. Another isolation procedure is the crystallization of the 2,3,5-trimethylhydroquinone dialkanoate from the mixture at the termination of the reaction by cooling, and, optionally, adding water, to the mixture to promote the crystallization.

The catalyst can be recovered by extraction with water or acid-water and concentration of the extract. Alternatively, the catalyst can be recovered by adding a biphasic solvent system, e. g. a carbonate (particularly ethylene carbonate or propylene carbonate) and an aliphatic hydrocarbon (particularly heptane or octane), and isolating it from the polar (carbonate) phase.

### Process for the manufacture of 2,3,5-trimethylhydroquinone

The 2,3,5-ttimethylhydroquinone dialkanoate obtained by the process of the present invention can be converted into 2,3,5-trimethylhydroquinone e.g. by transesterification, i.e. by treatment with an alcohol, e.g. an aliphatic alcohol such as isopropanol or n-butanol. Depending on the amounts of alcohol and catalyst and on the temperature in the reaction mixture, the transesterification yields the unesterified 2,3,5-trimethylhydroquinone and the ester formed as the further product Other processes known to the person skilled in the art may also be used for producing 2,3,5-trimethylhydroquinone from 2,3,5-trimethylhydroquinone dialkanoate. Such in a further aspect the invention also deals with a process for the manufacture of 2,3,5-trimethylhydroquinone wherein the 2,3,5-trimethylhydroquinone dialkanoate obtained by the process of the present invention is used as starting material.

### Process for the manufacture of α-tocopherol or its alkanoates

The former product, 2,3,5-trimethylhydroquinone, can be converted into e.g. (all-rac)-α-tocopherol by reaction with isophytol (and/or phytol), preferably in a biphasic solvent system, e.g. in a solvent system comprising a polar solvent such as ethylene or propylene carbonate, and an non-polar solvent, particularly an aliphatic hydrocarbon such as heptane. Other processes for the manufacture or α-tocopherol using 2,3,5-trimethylhydroquinone dialkanoate, 2,3,5-trimethylhydroquinone monoalkanoate or 2,3,5-trimethylhydroquinone as the intermediate or starting material are e.g. disclosed in US 4,808,736, EP-A 0 257 503, EP-A 0 269 009, US 5,283,346, US 4,208,334, EP-A 0 012 824, EP-A 0 782 993, US 5,900,494, WO 98/21197, US 5,908,939, EP-A 1 000 940, US 6,423,851, EP-A 1 134 218, US 6,369,242, WO 2004/046127 and WO 2004/063182. Thus, in a further aspect the invention deals with a process for the manufacture of α-tocopherol or its alkanoates, especially with a process for the manufacture of (all-*rac*)-α-tocopherol and/or its acetate, wherein 2,3,5-ttimethylhydroquinone dialkanoate obtained according to the process of the present invention is used as intermediate or starting material.

### Process for the manufacture of formulations of α-tocopherol or its alkanoates

The α-tocopherol or its alkanoate obtained by one of the processes disclosed in the documents mentioned above using a trimethylhydroquinone dialkanoate obtained by a process according to the present invention as intermediate or starting material can further be formulated by any method known to the person skilled in the art, e.g. as those disclosed in US 6,162,474, US 2001/0009679, US 6,180,130, US 6,426,078, US 6,030,645, US 6,150,086, US 6,146,825, US 6,001,554, US 5,938,990, US 6,530,684, US 6,536,940, US 2004/0053372, US 5,668,183, US 5,891,907, US 5,350,773, US 6,020,003, US 6,329,423, WO 96/32949, US 5,234,695, WO 00/27362, EP 0 664 116, US 2002/0127303, US 5,478,569, US 5,925,381, US 6,651,898, US 6,358,301, US 6,444,227, WO 96/01103 and WO 98/15195. Therefore, the present invention is also directed to a process for the manufacture of formulations of α-tocopherol and its alkanoates, especially of formulations of (all-*rac*)-α-tocopherol and its acetate, comprising the reaction of ketoisophorone to 2,3,5-trimethylhydroquinone dialkanoate according to the present invention as described above.

The invention is illustrated by the following Example.

### Example

A 230-ml four-necked flat-bottomed flask with heating/cooling jacket, equipped with a thermometer, a glass-tube for Ar-purge, a reflux condenser and a mechanical stirrer, was filled with the amount of catalyst given in the table below and 20.65 g (133 mmol) of ketoisophorone and a yellow solution was obtained, Within 10 minutes 40.64 g (400 mmol, 37.60 ml) of acetic anhydride were added under stirring. During addition, the mixture turned dark yellow to finally dark brown. The internal temperature was regulated by a thermostat. Samples were withdrawn and submitted to qualitative GC-analysis. The results are tabulated below :

| Catalyst (amount) [mol%] | Temperature [°C] | Reaction time [hours] | Conversion [%] | Purity [%] | | |
|---|---|---|---|---|---|---|
| | | | | KIP | TMHQ-DA | TMC-DA |
| In(SO₃CF₃)₃ (1) | 50 | 5 | 100.0 | 0.0 | 93.5 | 6.8 |
| In(SO₃ CF₃)₃^{a} (0.1) | 50 | 10^{a} | 65.3 | 34.7 | 58.5 | 4.1 |

| | | | | | | |
|---|---|---|---|---|---|---|
| KIP = ketoisophorone, TMHQ-DA = 2,3,6-trimethylhydroquinone diacetate; TMC-DA = 2,3,6-trimethyl catechol diacetate. The molar ratio of Ac₂O/KIP was 3/1. The amount of catalyst is based on KTP. ^{a} Inhibition of the reaction after the indicated time. | | | | | | |

## Claims

1. A process for the manufacture of a 2,3,5-trimethyhydroquinone dialkanoate comprising reacting ketoisophorone with an acylating agent in the presence of indium tris (trifluoromethanesulfonate) as a catalyst.

2. The process according to claim 1, wherein the acylating agent is an acid anhydride, an acyl halide or an enol ester.

3. The process according to claim 2, wherein the acylating agent is a straight or branched chain alkanoic acid anhydride, preferably acetic, propionic or butyric anhydride; a straight or branched chain alkanoyl chloride, preferably acetyl, propionyl or butyryl chloride; or, an enol ester, preferably isopropenyl acetate or butyrate.

4. The process according to one or more of claims 1 to 3, wherein the molar ratio of the acylating agent to ketoisophorone is from about 1 : 1 to about 5 : 1, preferably from about 2 : 1 to about 3:1, most preferably about 3 : 1.

5. The process according to one or more of claims 1 to 4, wherein the amount of the indium salt used as the catalyst is from about 0.1 mol-% to about 2 mol-%, preferably from about 0,1 to about 1 mol-%, based on the amount of ketoisophorone.

6. The process according to one or more of claims 1 to 5, wherein the acylating reaction is carried out at a temperature of from about 0°C to about 140°C, preferably from about 25°C to about 90°C, more preferably from about 25°C to about 70°C.

7. The process according to one or more of claims 1 to 6, wherein the 2,3,5-trimethylhydroquinone dialkanoate obtained is converted into (all-rac)-α-tocophelol by transesterification to yield 2,3,5-trimethylhydroquinone and reaction of the latter with isophytol and/or phytol.

8. A process for the manufacture of α-tocopherol and its alkanoates, especially of (all-*rac*)-α-tocopherol and its acetate, comprising the reaction of ketoisophorone to 2,3,5-trimethylhydroquinone dialkanoate according to one or more of claims 1 to 6.

9. A process for the manufacture of formulations of α-tocopherol and its alkanoates, especially of formulations of (all-*rac*)-α-tocopherol and its acetate, comprising the reaction of ketoisophorone to 2,3,5-trimethylhydroquinone dialkanoate according to one or more of claims 1 to 6.

## Patentansprüche

1. Verfahren zur Herstellung eines 2,3,5-Trimethylhydrochinondialkanoats, bei dem man Ketoisophoron in Gegenwart von Indiumtris(trifluormethansulfonat) als Katalysator mit einem Acylierungsmittel umsetzt.

2. Verfahren nach Anspruch 1, bei dem es sich bei dem Acylierungsmittel um ein Säureanhydrid, ein Acylhalogenid oder einen Enolester handelt.

3. Verfahren nach Anspruch 2, bei dem es sich bei dem Acylierungsmittel um ein gerad- oder verzweigtkettiges Alkansäureanhydrid, vorzugsweise Essigsäureanhydrid, Propionsäureanhydrid oder Buttersäureanhydrid; ein gerad- oder verzweigtkettiges Alkanoylchlorid, vorzugsweise Acetyl-, Propionyl- oder Butylylchlorid; oder einen Enolester, vorzugsweise Isopropenylacetat oder -butyrat handelt.

4. Verfahren nach einem oder mehreren der Ansprüche 1 bis 3, bei dem das Molverhältnis von Acylierungsmittel zu Ketoisophoron etwa 1 : 1 bis etwa 5 : 1, vorzugsweise etwa. 2 : 1 bis etwa 3 : 1, ganz besonders bevorsugt etwa 3 : 1, beträgt.

5. Verfahren nach einem oder mehreren der Ansprüche 1 bis 4, bei dem die Menge des als Katalysator verwendeten Indiumsalzes etwa 0,1 Mol-% bis etwa 2 Mol-%, vorzugsweise etwa 0,1 bis etwa 1 Mol-%, bezogen auf die Menge an Ketoisophoron, beträgt.

6. Verfahren nach einem oder mehreren der Ansprüche 1 bis 5, bei dem man die Acylierungsreaktion bei einer Temperatur von etwa 0°C bis etwa 140°C, vorzugsweise etwa 25°C bis etwa 90°C, weiter bevorzugt von etwa 25°C bis etwa 70°C, durchführt.

7. Verfahren nach einem oder mehreren der Ansprüche 1 bis 6, bei dem man das erhaltene 2,3,5-Trimethylhydrochinondialkanoat in (all-*rac*)-α-Tocopherol umwandelt, indem man es umestert, wobei man 2,3,5-Trimethylhydrochinon erhält, und dieses dann mit Isophytol und/oder Phytol umsetzt.

8. Verfahren zur Herstellung von α-Tocopherol und seinen Alkanoaten, insbesondere von (all-*rac*)-α-Tocopherol und seinem Acetat, bei dem man Ketoisophoron nach einem oder mehreren der Ansprüche 1 bis 6 zu 2,3,5-Trimethylhydrochinondialkanoat umsetzt.

9. Verfahren zur Herstellung von Formulierungen von α-Tocopherol und seinen Alkanoaten, insbesondere Formulierungen von (all-*rac*)-α-Tocopherol und seinem Acetat, bei dem man Ketoisophoron nach einem oder mehreren der Ansprüche 1 bis 6 zu 2,3,5-Trimethylhydrochinondialkanoat umsetzt.

## Revendications

1. Procédé de fabrication d'un dialcanoate de 2,3,5-triméthylhydroquinone comprenant la mise en réaction de cétoisophorone avec un agent d'acylation en présence de tris(trifluorométhanesulfonate) d'indium en tant que catalyseur.

2. Procédé selon la revendication 1, dans lequel l'agent d'acylation est un anhydride d'acide, un halogénure d'acyle ou un ester d'énol.

3. Procédé selon la revendication 2, dans lequel l'agent d'acylation est un anhydride d'acide alcanoïque linéaire ou ramifié, de préférence l'anhydride acétique, propionique ou butyrique ; un chlorure d'alcanoyle linéaire ou ramifié, de préférence le chlorure d'acétyle, de propionyle ou de butyryle ; ou un ester d'énol, de préférence l'acétate ou le butyrate d'isopropényle.

4. Procédé selon une ou plusieurs des revendications 1 à 3, dans lequel le rapport molaire entre l'agent d'acylation et la cétoisophorone est d'environ 1:1 à environ 5:1, de préférence d'environ 2:1 à environ 3:1, de manière préférée entre toutes d'environ 3:1.

5. Procédé selon une ou plusieurs des revendications 1 à 4, dans lequel la quantité du sel d'indium utilisé en tant que catalyseur est d'environ 0,1 % en moles à environ 2 % en moles, de préférence d'environ 0,1 à environ 1 % en moles, par rapport à la quantité de cétoisophorone.

6. Procédé selon une ou plusieurs des revendications 1 à 5, dans lequel la réaction d'acylation est réalisée à une température d'environ 0°C à environ 140 °C, de préférence d'environ 25 °C à environ 90 °C, de manière davantage préférée d'environ 25 °C à environ 70 °C.

7. Procédé selon une ou plusieurs des revendications 1 à 6, dans lequel le dialcanoate de 2,3,5-triméthylhydroquinone obtenu est transformé en (all-rac)-α-tocophérol par transestérification pour former la 2,3,5-triméthylhydroquinone et réaction de cette dernière avec de l'isophytol et/ou du phytol.

8. Procédé de fabrication d'α-tocophérol et ses alcanoates, notamment d'(all-rac)-α-tocophérol et son acétate, comprenant la transformation de cétoisophorone en dialcanoate de 2,3,5-triméthylhydroquinone selon une ou plusieurs des revendications 1 à 6.

9. Procédé de fabrication de formulations d'α-tocophérol et ses alcanoates, notamment de formulations d'(all-rac)-α-tocophérol et son acétate, comprenant la transformation de cétoisophorone en dialcanoate de 2,3,5-triméthylhydroquinone selon une ou plusieurs des revendications 1 à 6.
